# EUROPÄISCHE PATENTSCHRIFT

(11) **EP 0 752 451 B1**
(45) Veröffentlichungstag und Bekanntmachung des Hinweises auf die Patenterteilung: **17.10.2001**
(21) Anmeldenummer: 96109919.9
(22) Anmeldetag: 20.06.1996
(51) Int. Cl.: C09B 26/06, C09B 57/00, C09B 23/16, C07D 249/06

(54) **Die Verwendung von kationischen 4,5-Dihydro-1H-1,2,3-triazoliumverbindungen als Farbstoffe**
The use of 4,5-dihydro-1H-1,2,3-triazolium cationic compounds as dyestuffs
L'utilisation de composés 4,5-dihydro-1H-1,2,3-triazolium cationiques comme colorants

(30) Priorität: 03.07.1995 DE 19524133
(43) Veröffentlichungstag der Anmeldung: 08.01.1997
(73) Patentinhaber: BAYER AG, 51368 Leverkusen (DE)
(72) Erfinder: Böcker, Thomas, Dr., 42799 Leichlingen (DE); Berneth, Horst, Dr., 51373 Leverkusen (DE); Giera, Henry, Dr., 51429 Bergisch Gladbach (DE)

(56) Entgegenhaltungen:
- EP-A- 0 053 751
- US-A- 5 356 857
- CHEMISCHE BERICHTE, Bd. 112, 1979, Seiten 445-465, XP002015762 H. HANSEN ET AL: "Triazenium salze-eine neue Verbindungsklasse"
- 'The chemistry of synthetic dyes vol.4', 1971, VENKATARAMAN K., NEW YORK US XP002015763 * Seite 288, letzter Absatz - Seite 291, Absatz 1 *

## Beschreibung

Aus Chem. Ber. 112, 445 bis 461 (1979) und Liebigs Ann. Chem. 1980, 285 bis 290 sind einige Triazoliumverbindungen, sowie deren UV-Spektren und voltametrische Messung daran bekannt.

Es wurde nun gefunden, daß man 4,5-Dihydro-1H-1,2,3-triazoliumverbindungen der Formel (I) 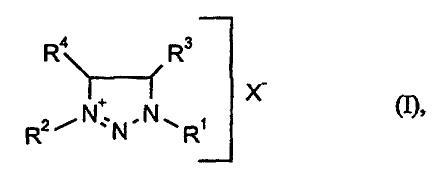 in der
- R¹ und R²: unabhängig voneinander C₆-C₁₄-Aryl oder einen heterocyclischen Rest mit bis zu 3 Ringen und bis zu 4 Heteroatomen aus der Reihe O, S und N bedeuten,
- R³ und R⁴: unabhängig voneinander Wasserstoff, C₁-C₁₂-Alkyl, C₂-C₁₂-Alkenyl, C₄-C ₈-Cycloalkyl, C₇-C₁₇-Aralkyl, C₆-C₁₄-Aryl oder Nitril bedeuten oder
- R³ und R⁴: gemeinsam eine 2- bis 5-gliedrige C-Brücke bedeuten, die gegebenenfalls von bis zu zwei Sauerstoff- und/oder Stickstoffatomen unterbrochen sein kann und
- X⁻: ein Anion bedeutet,
wobei alle vorhandenen Alkyl-, Alkenyl-, Cycloalkyl-, Aralkyl- und Arylreste, sowie annelierte und heterocyclische Reste gegebenenfalls mit nichtionogenen Substituenten, Carboxylgruppen, Ammoniumgruppen und/oder Pyridiniumgruppen substituiert sein können,
als Farbstoffe zum Färben und Bedrucken von kationisch färbbaren Fasern, vorzugsweise Polymerisaten und Mischpolymerisaten des Acrylnitrils und Dicyanoethylens, sowie von sauer modifizierten Fasern aus Polyamid und Polyester, wobei echte Farbtöne erhalten werden zum Färben und Bedrucken von tannierten Zellulosematerialien, Papier, Seide und Leder und zur Herstellung von Schreibflüssigkeiten, Stempelflüssigkeiten, Kugelschreiberpasten und ink-jet-Tinten und lassen sich auch im Gummidruck verwenden kann.

Alkylreste, auch solche in Alkoxy- und Aralkylresten können geradkettig oder verzweigt sein.

Nichtionogene Substituenten sind beispielsweise die in der Farbstoffchemie üblichen, nicht dissoziierenden Substituenten, wie Cyano, Hydroxy, Halogen, Nitro, C₁-C₁₂-Alkyl, C₁-C₁₂-Monoaikylamino, Di-C₁-C₁₂-alkylamino, C₆-C₁₂-Arylamino, C₇-C₁₅-Aralkylamino, C₁-C₁₂-Alkoxy, Phenyl, C₁-C₁₂-Acylamino, C₁-C₁₂-Alkoxycarbonyl, C₁-C₁₂-Alkoxycarbonyloxy, C₁-C₁₂-Alkamidocarbonyl, C₁-C₁₂-Alkylsulfonyl, C₆-C₁₀-Arylazo, Aminocarbonyl, Phenylsulfonyl, Sulfonamid, Sulfonamyl und Ureido, die ihrerseits gegebenenfalls mit Hydroxy, Halogen, Cyano und/oder C₁-C₆-Alkoxy substituiert sein können.

X⁻ steht vorzugsweise für ein farbloses, organisches oder anorganisches Anion, beispielsweise Fluorid, Chlorid, Bromid, Iodid, Perchlorat, Tetrafluoroborat, Hydroxid, Hydrogensulfat, Sulfat, Dihydrogenphosphat, Hydrogenphosphat, Phosphat, Hydrogencarbonat, Carbonat, Methylsulfat, Ethylsulfat, Cyanat, Thiocyanat, Tri- oder Tetrachlorozinkat, Tetrachloroferrat, Hexafluorosilikat oder ein Anion einer gesättigten oder ungesättigten aliphatischen, cycloaliphatischen, aromatischen oder heterocyclischen Carbon- oder Sulfonsäure beispielsweise Formiat, Acetat, Hydroxyacetat, Cyanacetat, Propionat, Hydroxypropionat, Oxalat, Citrat, Lactat, Tartrat, das Anion der Cyclohexancarbonsäure, Phenylacetat, Benzoat, das Anion der Nikotinsäure, Methansulfonat, Ethansulfonat, Benzolsulfonat, Chlorbenzolsulfonat und Toluolsulfonat.

Wenn es sich um ein mehrwertiges Anion handelt, z.B. um Sulfat oder Oxalat, dann steht in Formel (I) X⁻ für ein Äquivalent solch eines mehrwertigen Anions.

Erfindungsgemäß werden bevorzugt 4,5-Dihydro-1H-1,2,3-triazoliumverbindungen der Formel (I) als Farbstoffe verwendet, bei denen
- R¹ und R²: unabhängig voneinander Phenyl, Naphthyl, Thienyl, Thiazolyl, Isothiazolyl, 1,2,4-Thiadiazolyl, 1,3,4-Thiadiazolyl, Benzthiazolyl, Benzisothiazolyl, Pyrazolyl, Imidazolyl, Benzimidazolyl, Oxazolyl, Benzoxazolyl, 1,2,3-Triazolyl, 1,2,4-Triazolyl, Indolyl oder Pyridyl bedeuten, wobei diese Reste gegebenenfalls mit bis zu 5 Resten aus der Gruppe C₁-C₈-Alkyl, C₁-C₈-Alkoxy, C₆-C₁₀-Aryloxy, C₆-C₁₀-Arylamino, C₇-C₁₂-Aralkylamino, C₁-C₈-Acylamino, C₁-C₈-Acyloxy, C₁-C₈-Monoalkylamino, Di-C₁-C₈-alkylamino, C₁-C₈-Alkoxycarbonyl, C₁-C₈-Alkoxycarbonyloxy, C₁-C₁₂-Alkamidocarbonyl, C₁-C₁₂-Alkylsulfonyl, C₆-C₁₀-Arylazo, Aminocarbonyl, Halogen, Cyano, Hydroxy, Nitro, Phenylsulfonyl, Sulfonamid, Sulfonamyl und Ureido substituiert sein können, die ihrerseits gegebenenfalls mit Hydroxy, Halogen, Cyano und/oder C₁-C₄-Alkoxy substituiert sein können,
R³ und R⁴ unabhängig voneinander jeweils Wasserstoff, gegebenenfalls durch Hydroxy, Halogen, Cyano, C₁-C₄-Alkoxy, Aminocarbonyl und/oder C₁-C₄-Alkoxycarbonyl substituiertes C₁-C₈-Alkyl, Allyl, Cyclopentyl oder Cyclohexyl, einen gegebenenfalls durch Halogen, Cyano, C₁-C₄-Alkyl und/oder C₁-C₄-Alkoxy substituierten Benzyl-, Phenethyl-, Furyl-, Tetrahydrofurylmethyl-, Pyridyl-, Pyridylmethyl- oder Pyridylethylrest oder einen gegebenenfalls durch Halogen, Cyano, C₁-C₄-Alkyl oder C₁-C₄-Alkoxy substituierten Phenylrest bedeuten oder
R³ und R⁴ gemeinsam mit den dazwischenliegenden zwei Kohlenstoffatomen einen gegebenenfalls durch Hydroxy, Halogen, Cyano, C₁-C₄-Alkoxy und/oder Aminocarbonyl substituierten Cyclobutan-, Cyclopentan-, Cyclohexan- oder Cycloheptanring der gegebenenfalls von einem Stickstoffatom unterbrochen ist, einen gegebenenfalls durch Hydroxy, Halogen, Cyano, C₁-C₄-Alkoxy und/oder Aminocarbonyl substituierten Tetrahydropyrrolidino- oder Piperidinoring, der gegebenenfalls von einem Sauerstoffatom unterbrochen ist oder einen gegebenenfalls durch Hydroxy, Halogen, Cyano, C₁-C₄-Alkoxy und/oder Aminocarbonyl substituierten Tetrahydrofuryl- oder Tetrahydropyranylring bilden und
X⁻ ein Anion bedeutet.

Halogen steht vorzugsweise für Fluor, Chlor und Brom.

Erfindungsgemäß werden besonders bevorzugt 4,5-Dihydro-1H-1,2,3-triazoliumverbindungen der Formel (I) als Farbstoffe verwendet, in denen
- R¹ und R²: unabhängig voneinander Phenyl, Naphthyl, Thienyl, Thiazolyl, Isothiazolyl, 1,2,4-Thiadiazolyl, 1,3,4-Thiadiazolyl, Benzthiazolyl, Benzisothiazolyl, Imidazolyl, Benzimidazolyl, Benzoxazolyl, 1,2,4-Triazolyl oder Pyridyl bedeuten, wobei diese Reste gegebenenfalls mit bis zu 5 Resten aus der Gruppe C₁-C₄-Alkyl, C₁-C₄-Alkoxy, C₆-C₁₀-Aryloxy, C₆-C₁₀-Arylamino, C₇-C₁₂-Aralkylamino, C₁-C₄-Acylamino, C₁-C₄-Acyloxy, C₁-C₄-Monoalkylamino, Di-C₁-C₄-alkylamino, C₁-C₄-Alkoxycarbonyl, C₁-C₄-Alkoxycarbonyloxy, C₁-C₄-Alkamidocarbonyl, C₁-C₄-Alkylsulfonyl, C₆-Arylazo, Aminocarbonyl, Halogen, Cyano, Hydroxy, Nitro, Phenylsulfonyl, Sulfonamid, Sulfonylamyl und Ureido substituiert sein können, die ihrerseits gegebenenfalls mit Hydroxy, Halogen, Cyano und/oder C₁-C₄-Alkoxy substituiert sein können,
- R³ und R⁴: unabhängig voneinander jeweils Wasserstoff oder einen gegebenenfalls durch Hydroxy, Halogen, Cyano, C₁-C₄-Alkoxy, Aminocarbonyl und/oder C₁-C₄-Alkoxycarbonyl substituierten C₁-C₄-Alkylrest bedeuten oder
- R³ und R⁴: gemeinsam mit den dazwischenliegenden zwei Kohlenstoffatomen einen gegebenenfalls durch Hydroxy, Halogen, Cyano, C₁-C₄-Alkoxy und/oder Aminocarbonyl substituierten Cyclopentan- oder Cyclohexanring bedeuten und
- X⁻: ein Anion bedeutet.

Erfindungsgemäß werden ganz besonders bevorzugt 4,5-Dihydro-1H-1,2,3-triazoliumverbindungen der Formel (I) als Farbstoffe verwendet, die der Formel (II) entsprechen 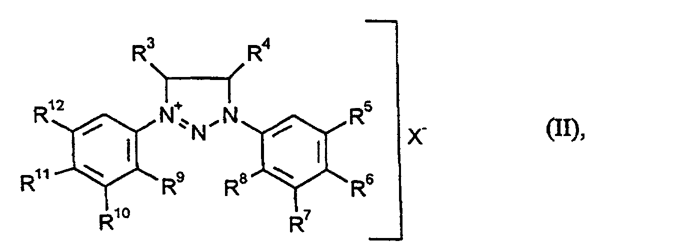 in der
- R³ und R⁴: unabhängig voneinander jeweils Wasserstoff oder einen gegebenenfalls durch Hydroxy, Halogen, Cyano, C₁-C₄-Alkoxy, Aminocarbonyl und/oder C₁-C₄-Alkoxycarbonyl substituierten C₁-C₄-Alkylrest bedeuten oder
- R³ und R⁴: gemeinsam mit den dazwischenliegenden zwei Kohlenstoffatomen einen gegebenenfalls durch Hydroxy, Halogen, Cyano, C₁-C₄-Alkoxy und/oder Aminocarbonyl substituierten Cyclopentan- oder Cyclohexanring bedeuten,
- R⁵ bis R¹²: unabhängig voneinander jeweils Wasserstoff oder einen gegebenenfalls durch Hydroxy, Halogen, Cyano und/oder C₁-C₄-Alkoxy substituierten C₁-C₄-Alkyl-, C₁-C₄-Alkoxy-, C₆-C₁₀-Aryloxy-, C₆-C₁₀-Arylamino-, C₇-C₁₂-Aralkylamino-, C₁-C₄-Acylamino-, C₁-C₄-Acyloxy-, C₁-C₄-Monoalkylamino-, Di-C₁-C₄-alkylamino, C₁-C₄-Alkoxycarbonyl-, C₁-C₄-Alkoxycarbonyloxy-, C₁-C₄-Alkamidocarbonyl-, C₁-C₄-Alkylsulfonyl- oder C₆-Arylazo-Rest, Aminocarbonyl, Halogen, Cyano, Hydroxy, Nitro, Phenylsulfonyl, Sulfonamid, Sulfonamyl oder Ureido bedeuten und
- X⁻: ein Anion bedeutet.

Erfindungsgemäß werden bevorzugt solche 4,5-Dihydro-1H-1,2,3-triazoliumverbindungen der Formel (II) als Farbstoffe verwendet, bei denen
- R³ und R⁴: unabhängig voneinander Wasserstoff oder einen C₁-C₄-Alkylrest bedeuten,
- R⁵ bis R⁷ und R¹⁰ bis R¹²: unabhängig voneinander jeweils Wasserstoff, einen C₁-C₄-Alkyl-, C₁-C₄-Alkoxy-, Phenoxy-, Phenylamino-, Phenyl-C₁-C₄-alkylamino-, C₁-C₄-Acylamino-, C₁-C₄-Acyloxy-, C₁-C₄-Monoalkylamino-, Di-C₁-C₄-dialkylamino-, C₁-C₄-Alkoxycarbonyl-, C₁-C₄-Alkoxycarbonyloxy-, C₁-C₄-Alkamidocarbonyl-, C₁-C₄-Alkylsulfonyl- oder Phenylazo-Rest, Aminocarbonyl, Halogen, Cyano, Hydroxy, Nitro, Phenylsulfonyl, Sulfonamid, Sulfonamyl oder Ureido bedeuten,
- R⁸ und R⁹: unabhängig voneinander jeweils Wasserstoff, C₁-C₄-Alkyl, C₁-C₄-Alkoxy, Halogen, Cyano oder Hydroxy bedeuten und
- X⁻: ein Anion bedeutet.

Erfindungsgemäß werden besonders bevorzugt solche 4,5-Dihydro-1H-1,2,3-triazoliumverbindungen der Formel (II) als Farbstoffe verwendet, bei denen
- R³ und R⁴: unabhängig voneinander Wasserstoff oder Methyl bedeuten,
- R⁵ und R¹², R⁶ und R¹¹, R⁷ und R¹⁰, R⁸ und R⁹: jeweils paarweise gleich sind und eine der oben bei Formel (II) als bevorzugt angegebenen Bedeutungen haben und
- X⁻: ein Anion bedeutet.

Erfindungsgemäß werden ganz besonders bevorzugt solche 4,5-Dihydro-1H-1,2,3-triazoliumverbindungen der Formel (II) als Farbstoffe verwendet, bei denen
- R³ und R⁴: unabhängig voneinander Wasserstoff oder Methyl bedeuten,
- R⁶: Wasserstoff, Methyl, Methoxy, Ethoxy, Phenoxy, Acetylamino, Dimethylamino, Diethylamino, Methoxycarbonyl, Ethoxycarbonyl, Aminocarbonyl, Chlor, Cyano oder Nitro bedeutet,
für den Fall, daß R³ und R⁴ jeweils Wasserstoff bedeuten,
- R¹¹: Ethoxy, Phenoxy, Acetylamino, Dimethylamino, Diethylamino, Methoxycarbonyl, Ethoxycarbonyl, Aminocarbonyl, Chlor, Cyano oder Nitro bedeutet,
für den Fall, daß mindestens einer der Reste R³ und R⁴ von Wasserstoff verschieden ist
- R¹¹: zusätzlich auch Wasserstoff, Methyl, Methoxy oder Nitro bedeuten kann,
- R⁵ und R¹² und R⁷ und R¹⁰: jeweils paarweise gleich sind und Wasserstoff, Methyl, Methoxy, Ethoxy, Phenoxy, Acetylamino, Dimethylamino, Diethylamino, Methoxycarbonyl, Ethoxycarbonyl, Aminocarbonyl, Chlor, Cyano oder Nitro bedeuten,
- R⁸ und R⁹: unabhängig voneinander jeweils Wasserstoff, Methyl oder Methoxy bedeuten und
- X⁻: ein Anion bedeutet.

Weiterhin werden erfindungsgemäß ganz besonders bevorzugt 4,5-Dihydro-1H-1,2,3-triazoliumverbindungen der Formel (II) als Farbstoffe verwendet, bei denen
- R³, R⁵, R⁸, R⁹ und R¹²: jeweils Wasserstoff bedeuten,
- R⁴: Wasserstoff oder Methyl bedeutet,
- R⁶: Methyl, Methoxy, Ethoxy, Phenoxy, Acetylamino, Dimethylamino, Diethylamino, Methoxycarbonyl, Ethoxycarbonyl oder Aminocarbonyl bedeutet,
für den Fall, daß R⁴ Wasserstoff bedeutet,
- R¹¹: Ethoxy, Phenoxy, Acetylamino, Dimethylamino, Diethylamino, Methoxycarbonyl, Ethoxycarbonyl oder Aminocarbonyl bedeutet,
für den Fall, daß R⁴ Methyl bedeutet,
- R¹¹: zusätzlich auch Wasserstoff, Methyl, Methoxy oder Nitro bedeuten kann,
- R⁷ und R¹⁰: unabhängig voneinander jeweils Wasserstoff, Methyl oder Methoxy bedeuten und
- X⁻: ein Anion bedeutet.

Die Herstellung von 4,5-Dihydro-1H-1,2,3-triazoliumverbindungen der Formel (I) kann z.B. erfolgen, indem man eine Diazokomponente der Formel (III)

R¹-NH₂ (III),

in der
- R¹: die bei Formel (I) angegebene breiteste Bedeutung hat,
auf das sekundäre Stickstoffatom einer aktivierten Kupplungskomponente der Formel (VI) kuppelt

R²-NH-CHR³-CHR⁴R¹³ (VI),

in der
- R², R³ und R⁴: die bei Formel (I) angegebene breiteste Bedeutung haben und
- R¹³: eine Abgangsgruppe bedeutet,
und dann die entstandenen Triazene der Formel (VII)

R¹-N=N-N(-CHR³-CHR⁴R¹³)-R² (VII),

in der
R¹, R², R³, R⁴ und R¹³ die oben angegebene Bedeutung haben,
zu 4,5-Dihydro-1H-1,2,3-triazoliumverbindungen der Formel (I) cyclisiert.

Bei der Abgangsgruppe R¹³ kann es sich z.B. um Methansulfonat, Chlorid, Bromid, Benzolsulfonat, p-Toluolsulfonat, Trifluoracetat, Acetat oder Sulfat handeln.

Die aktivierten Kupplungskomponenten der Formel (VI) sind auf an sich bekannte Weise erhältlich, beispielsweise durch Umsetzung von Verbindungen der Formel (IV)

R²-NH-CHR³-CHR⁴-OH (IV),

in der
R², R³ und R⁴ die bei Formel (I) angegebene breiteste Bedeutung haben,
mit einem Säurehalogenid, einem Säureanhydrid oder einer Mineralsäure, z.B. mit Methansulfonsäurechlorid, Benzolsulfonsäurechlorid, p-Toluolsulfonsäurechlorid, Trifluoressigsäurechlorid, Trifluoressigsäureanhydrid, Acetanhydrid, Acetylchlorid, Bromwasserstoff, Chlorwasserstoff oder Schwefelsäure.

Die aromatischen und heterocyclischen Diazokomponenten der Formeln (III) und aromatische und heterocyclische Kupplungskomponenten der Formeln (IV) sind bekannt z.B. aus Houben-Weyl, Methoden der Organischen Chemie, Band XI/1, Seiten 3 bis 262 und 341 bis 1025 oder analog dazu erhältlich.

Die Aktivierung der Hydroxylgruppe kann in an sich bekannter Weise durchgeführt werden, beispielsweise durch Umsetzung der Hydroxylgruppe mit einem Säurehalogenid oder Anhydrid, z.B. mit Methansulfonsäurechlorid, Benzolsulfonsäurechlorid, p-Toluolsulfonsäurechlorid, Trifluoressigsäurechlorid, Trifluoressigsäureanhydrid, Acetanhydrid oder Acetylchlorid.

Die Herstellung von 4,5-Dihydro-1H-1,2,3-triazoliumverbindungen der Formel (I) kann z.B. auch erfolgen, indem man eine Diazokomponente der Formel (III) diazotiert, auf das Stickstoffatom einer Kupplungskomponente der Formel (VIII) kuppelt

R²-NH₂ (VIII),

in der
- R²: die bei Formel (I) angegebene breiteste Bedeutung hat und
das entstandene Triazen der Formel (IX)

R¹-N=N-NH-R² (IX),

in der
R¹ und R² die oben angegebene Bedeutung haben,
mit Verbindungen der Formel (X)

R¹⁵-CHR³-CHR⁴-R¹⁴ (X),

in der
- R³ und R⁴: die bei Formel (I) angegebene breiteste Bedeutung haben,
- R¹⁴: für eine nucleophil zu substituierende Abgangsgruppe steht und
- R¹⁵: für Hydroxy oder eine Abgangsgruppe steht,
umsetzt und anschließend cyclisiert, im Falle von R¹⁵ = Hydroxy nach wie oben beschriebener vorhergehender Aktivierung.

R¹⁴ und R¹⁵, letzteres soweit es eine Abgangsgruppe bedeutet, können beispielsweise von der gleichen Art sein, wie für R¹³ angegeben.

Die erwähnten Diazotierungen können in an sich bekannter Weise durchgeführt werden, beispielsweise nach Houben-Weyl, Methoden der Organischen Chemie, Band X/3, Seiten 1 bis 112 und E16a, Seiten 1052 bis 1087. Hierzu verwendet werden können z.B. Natriumnitrit in wäßriger Mineralsäure, z.B. Salzsäure oder Schwefelsäure oder Nitrosylschwefelsäure in 80 bis 90 gew.-%iger Phosphorsäure oder in Gemischen solcher Phosphorsäuren mit Essigsäure, Propionsäure und/oder Schwefelsäure.

Die erwähnten Kupplungen zu den Triazenen der Formel (V), (VII) und (IX) können ebenfalls auf an sich bekannte Weise durchgeführt werden, beispielsweise nach Houben-Weyl, Methoden der Organischen Chemie, Band X/3, Seiten 695 bis 723 und E16a, Seiten 1186 bis 1220.

Die Diazotierungen und Kupplungen können auch nach anderen an sich bekannten Verfahren simultan durchgeführt werden, beispielsweise indem man Verbindungen der Formeln (III) und (IIIa) und Verbindungen der Formeln (IV) oder (IVa) gemeinsam in einem sauren Medium mit beispielsweise Natriumnitrit umsetzt. Als saure Medien sind z.B. wäßrige Mineralsäuren oder organische Säuren oder Mischungen davon geeignet, wobei als wäßrige Mineralsäuren z.B. wäßrige Salzsäure, Schwefelsäure oder Phosphorsäure und als organische Säuren z.B. Ameisensäure, Essigsäure oder Propionsäure in Frage kommen. Auch unter Druck verflüssigtes Kohlendioxid kann als saures Medium dienen.

Die entstehenden Verbindungen der Formeln (I) fallen aus den Lösungsmitteln direkt aus und können, z.B. durch Abfiltrieren, isoliert werden. Man kann sie bei der Verwendung von mit Wasser mischbaren Lösungsmitteln als feste, abfiltrierbare Produkte erhalten durch Verdünnen mit Wasser und Zugabe wasserlöslicher Salze wie Natrium- oder Kaliumchlorid, gegebenenfalls in Gegenwart von Zinkchlorid. Hochreine Verbindungen können z.B. durch chromatographische Reinigung an Kieselgel erhalten werden.

Das Färben von beispielsweise Polymerisaten und Mischpolymerisaten des Acrylnitrils kann z.B. aus schwach saurer Flotte erfolgen, wobei man in das Färbebad vorzugsweise bei 40 bis 60°C eingeht und dann bei Kochtemperatur färbt. Man kann auch unter Druck bei Temperaturen über 100°C färben. Des weiteren lassen sich die Verbindungen der Formeln (I) in Spinnlösungen zur Herstellung von gefärbten Fasern einbringen.

Die Färbungen mit Verbindungen der Formeln (I) auf Materialien aus Polyacrylnitril zeichnen sich durch sehr gute Licht-, Naß- und Reibeechtheiten und durch eine hohe Affinität zur Faser aus.

Verbindungen der Formeln (I) können einzeln, in Mischungen untereinander oder in Mischungen mit anderen Farbstoffen angewendet werden.

Bevorzugt, besonders bevorzugt und ganz besonders bevorzugt werden solche Verbindungen der Formeln (I) hergestellt und zum Färben verwendet, die bei der Beschreibung der Verbindungen so bezeichnet sind.

### Beispiele

### Beispiel 1

2,3 g 3,4-Dimethoxyanilin wurden in einer Mischung aus 22 ml Wasser und 3,75 ml konzentrierter Salzsäure bei 0 bis 5°C durch Zutropfen von 1,1 g Natriumnitrit, gelöst in 4 ml Wasser, diazotiert. Die Diazoniumsalzlösung tropfte während 15 min bei 0 bis 5°C zu einer Lösung von 2,29 g der Kupplungskomponente der Formel (IV) mit R² = p-Phenoxyphenyl und R³ = R⁴ = Wasserstoff in 50 ml Methanol, 50 ml Eis/Wasser und 9 g Natriumhydrogencarbonat. Nach erfolgter Zugabe wurde 10 min nachgerührt und sofort mit Methylenchlorid extrahiert. Die organische Phase wurde mit Natriumsulfat getrocknet. Das in der organischen Phase enthaltene Triazen der Formel (V) mit R¹ = 3,4-Dimethoxyphenyl, R² = p-Phenoxyphenyl und R³ = R⁴ = Wasserstoff wurde anschließend direkt mit Methansulfonsäure cyclisiert. Dazu wurde die organische Phase mit 1,68 g Diazabicyclononan (DABCO) versetzt und bei -10°C langsam 1,16 ml Methansulfonsäurechlorid, gelöst in 3 ml Methylenchlorid zugetropft. Nach 60 min Reaktionszeit wurde die Lösung eingeengt. Nach chromatographischer Reinigung an Kieselgel mit dem Laufmittelgemisch Methylenchlorid:Methanol 4:1 wurden 2,1 g (52 %) eines orange-gelben Pulvers erhalten. Das Produkt der Formel (II) mit R³, R⁴, R⁵, R⁷, R⁸, R⁹ und R¹² = Wasserstoff, R⁶ =Phenoxy, R¹⁰ = R¹¹ = Methoxy und X = Cl ergab in Methanol ein λₘₐₓ von 431 nm.
Fab-MS: 376 (M⁺).
- ¹H-NMR (CD₃OD):: 2,69, 3,92, 3,97, 5,03, 7.08, 7,15, 7,18, 7,22, 7,28, 7,38, 7,43 und 7,78 ppm.

### Beispiel 2

2,25 g p-Aminoacetanilid wurden in einer Mischung aus 22 ml Wasser und 3,75 ml konzentrierter Salzsäure bei 0 bis 5°C durch Zutropfen von 1,1 g Natriumnitrit, gelöst in 4 ml Wasser diazotiert. Die Diazoniumsalzlösung tropfte während 15 min bei 0 bis 5°C zu einer Lösung von 1,67 g der Kupplungskomponente der Formel (IV) mit R² = p-Methoxy und R³ = R⁴ = Wasserstoff in 50 ml Methanol, 50 ml Eis/Wasser und 9 g Natriumhydrogencarbonat. Nach erfolgter Zugabe wurde 10 min nachgerührt und anschließend abgesaugt. Das isolierte Triazen der Formel (V) mit R¹ = p-Acetaminophenyl, R² = p-Methoxyphenyl und R³ = R⁴ = Wasserstoff wurde anschließend in Acetonitril gelöst und analog Beispiel 1 cyclisiert. Der ausgefallene Niederschlag wurde abgesaugt. Nach chromatographischer Reinigung an Kieselgel mit dem Laufmittelgemisch Methylenchlorid:Methanol 4:1 wurden 2,4 g (69 %) eines orangen Pulvers erhalten. Das Produkt der Formel (II) mit R³, R⁴, R⁵, R⁷, R⁸, R⁹, R¹⁰ und R¹² = Wasserstoff, R⁶ = Methoxy, R¹¹ = Acetamino und X = Cl ergab in Methanol ein λₘₐₓ von 426 nm.
Fab-MS: 311 (M⁺).
¹H-NMR (CD₃OD): 2,16, 2,69, 3,90, 5,01, 7,17 und 7,70 ppm.

### Beispiel 3

9 g p-Anisidin wurden in einer Mischung aus 50 ml Wasser und 20 ml konzentrierter Salzsäure bei 0 bis 5°C durch Zutropfen von 18 ml 30 gew.-%iger Natriumnitritlösung diazotiert. Diese Diazoniumsalzlösung tropfte während 30 min bei 0 bis 5°C zu einer Lösung von 20,4 g des Hydrobromids der aktivierten Kupplungskomponente der Formel (VI) mit R² = Phenyl, R³ = R⁴ = Wasserstoff und R¹³ = Brom in 125 ml Methanol, 125 ml Wasser und 46 g Natriumhydrogencarbonat. Nach Rühren über Nacht bei Raumtemperatur wurde abgesaugt und mit 200 ml Wasser gewaschen. Die gelbe Mutterlauge wurde mit 60 ml 2-molarer wäßriger Zinkchloridlösung versetzt und der Niederschlag abgesaugt und getrocknet. Man erhielt 7,4 g (24 % der Theorie) Pulver der Formel (II) mit R⁶ = Methoxy, R³ bis R⁵ und R⁷ bis R¹² = Wasserstoff und X⁻ = ZnCl₃⁻. In Ethanol/Eisessig ergab sich ein λₘₐₓ von 407,5 nm.
¹H-NMR (CD₃OD): 3,90, 5,09, 7,08, 7,52, 7,63 und 7,77 ppm.

Die aktivierte Kupplungskomponente der Formel (VI) wurde folgendermaßen hergestellt:

Zu 100 g N-(2-Hydroxyethyl)-anilin tropften bei 0°C 310 ml 48 gew.-%ige Bromwasserstoffsäure. Anschließend wurde zum Sieden erhitzt und über eine Füllkörperkolonne solange abdestilliert, bis die Übergangstemperatur 125°C erreicht hatte. Bei dieser Übergangstemperatur wurden insgesamt 347 g Destillat abdestilliert. Dann wurde auf Raumtemperatur abgekühlt, mit 500 ml Aceton verdünnt und abgesaugt. Man erhielt nach dem Trocknen 103 g (50 % d.Th.) des Hydrobromids der aktivierten Kupplungskomponente der Formel (VI), wobei die Reste die oben für die aktive Kupplungskomponente genannte Bedeutung hatten, als farbloses Kristallisat vom Schmelzpunkt 135 bis 137°C.

### Beispiel 4

Es wurde wie in Beispiel 3 verfahren, jedoch wurden 17,9 g der aktivierten Kupplungskomponente der Formel (VI) mit R² = 4-Methoxyphenyl, R³ = R⁴ = Wasserstoff und R¹³ = -O-SO₂-OH eingesetzt. Man erhielt 13,2 g (40 % d.Th.) gelbes Pulver der Formel (II) mit R⁶ = R¹¹ = Methoxy, R³ bis R⁵, R⁷ bis R¹⁰ und R¹² = Wasserstoff und X⁻ = ZnCl₃⁻. In Methanol ergab sich ein λₘₐₓ von 423 nm.
Fab-MS: 284 (M⁺).
¹H-NMR (CD₃OD): 2,69, 3,90, 4,99, 7,17 und 7,72 ppm.

Die aktivierte Kupplungskomponente der Formel (VI) wurde folgendermaßen hergestellt:

Bei 0°C wurden 4,5 g N-(2-Hydroxyethyl)-4-methoxyanilin in 8 ml konzentrierter Schwefelsäure eingetragen. 24 h wurde bei 0°C gerührt und dann auf 150 g Eis ausgetragen. Mit 8 ml 45 gew.-%iger wäßriger Natronlauge wurde die Lösung auf pH = 4,5 gestellt. Nach Rühren über Nacht wurde abgesaugt. Man erhielt 4,4 g (66 % d.Th.) der aktivierten Kupplungskomponente der Formel (VI), wobei die Reste die oben für die aktive Kupplungskomponente genannte Bedeutung besaßen, als farbloses Kristallisat. Im Massenspektrum (FAB) wird die Masse 247 gefunden.

### Beispiel 5

61,6 g p-Anisidin wurden in 170 g Eis und 250 ml Wasser vorgelegt und mit 76,5 g konzentrierter Salzsäure versetzt. Bei 0 bis 5°C tropfte eine Lösung von 17,4 g Natriumnitrit in 42 ml Wasser innerhalb von 15 Minuten zu. In 10 Minuten tropfte eine weitere Lösung von 70,5 g Natriumacetat in 135 ml Wasser zu. Nach 2 Stunden Rühren bei Raumtemperatur wurde abgesaugt und mit Eiswasser gewaschen. Es verblieben nach Trocknen 50,4 g (78 %) gelbes Pulver des Triazens der Formel (IX) mit R¹ = R² = p-Methoxyphenyl.

2,6 g Triazen der Formel (IX) mit R¹ = R² = p-Methoxyphenyl wurden in 25 ml tert.-Butanol mit 2,3 g Kalium tert.-Butylat 15 Minuten zum Rückfluß erhitzt.

Nach Zutropfen von 1,6 g Chlorethanol wurde weitere zwei Stunden erhitzt. Nach Abkühlen auf 10°C tropfte man nacheinander 3,5 g Benzolsulfochlorid und 2,8 ml Triethylamin zu und rührte 2 Stunden bei Raumtemperatur. Nach Absaugen und Trocknen erhielt man aus der orangefarbenen Suspension 2,8 g (63 %) gelb-oranges Pulver der Formel (II) mit R³, R⁴, R⁵, R⁷, R⁸, R⁹, R¹⁰ und R¹² = Wasserstoff, R⁶ = R¹¹ = Methoxy und X = Benzolsulfonat.

Die analytischen Daten entsprechen den in Beispiel 4 angegebenen.

### Beispiele 6 bis 97

Es wurde analog den Beispielen 1 bis 5 verfahren und so folgende Farbstoffe der Formel (I) erhalten, wobei R³ und R⁴, soweit nichts anderes angegeben ist Wasserstoff und X⁻ soweit nichts anderes angegeben ist Chlorid bedeuten.

| Beispiel Nr. | analog Beispiel Nr. | R¹ | R² | λₘₐₓ (nm) | Fab-MS (M⁺) |
|---|---|---|---|---|---|
| 6 | 1 | 4-Nitrophenyl | 4-Chlorphenyl | 397 | 304 |
| 7 | 1 | 4-Methoxyphenyl | 4-Cyanophenyl | 420 | 279 |
| 8 | 1 | 3,4-Dichlorophenyl | 4-Phenoxyphenyl | 410 | 385 |
| 9 | 1 | 3,5-Dimethoxyphenyl | 3,4-Dichlorophenyl | 395 | 353 |
| 10 | 1 | 4-Phenoxyphenyl | 4-Chlorophenyl | 409 | 351 |
| 11 | 1 | 4-Phenoxyphenyl | 4-Tolyl | 408 | 331 |
| 12 | 1 | 4-Methoxyphenyl | 4-Chlorophenyl | 415 | 288 |
| 13 | 1 | 4-Phenoxyphenyl | 4-Chlorophenyl | 409 | 351 |
| 14 | 1 | 4-Nitrophenyl | 4-Methoxyphenyl | 426 | 299 |
| 15 | 1 | 4-Nitrophenyl | 4-Phenoxyphenyl | 418 | 361 |
| 16 | 1 | 2,4-Dimethoxyphenyl | 4-Cyanophenyl | 415 | 309 |
| 17 | 1 | 4-Cyanophenyl | 4-Tolyl | 397 | 263 |
| 18 | 1 | 4-Phenoxyphenyl | 4-Cyanophenyl | 412 | 341 |
| 19 | 1 | 2,4-Dimethoxyphenyl | 3,4-Dichlorophenyl | 409 | 353 |
| 20 | 1 | 3,4-Dichlorophenyl | 4-Methoxyphenyl | 416 | 323 |
| 21 | 1 | 2,4-Dichlorophenyl | 4-Tolyl | 397 | 307 |
| 22 | 1 | 2,4-Dimethoxyphenyl | 2-Methyl-4-nitrophenyl | 403 | 343 |
| 23 | 1 | 2-Methyl-4-nitrophenyl | 4-Methoxyphenyl | 409 | 313 |
| 24 | 1 | 2-Methyl-4-nitrophenyl | 4-Tolyl | 388 | 297 |
| 25 | 1 | 2-(5-Diisopropylamino-1,3,4-thiadiazolyl) | 4-Methoxyphenyl | 474 | 364 |
| 26 | 1 | wie bei Beispiel 25 | 4-Tolyl | 467 | 348 |
| 27 | 1 | wie bei Beispiel 25 | 2,4-Dimethoxyphenyl | 462 | 390 |
| 28 | 1 | wie bei Beispiel 25 | 4-Phenoxyphenyl | 474 | 424 |
| 29 | 1 | 2,4,6-Trimethylphenyl | (5-Diisopropylamino-1,3,4-thiadiazol)-2-yl | 432 | 374 |
| 30 | 1 | 2,4-Dimethoxyphenyl | 4-Methoxyphenyl | 406 | 314 |
| 31 | 1 | 4-Methoxyphenyl | 4-Methoxyphenyl | 424 | 284 |
| 32 | 1 | 2,4-Dimethoxyphenyl | 4-Nitrophenyl | 422 | 329 |
| 33 | 1 | 2,4-Dimethoxyphenyl | 4-Chlorophenyl | 403 | 319 |
| 34 | 1 | R¹ und R² wie in Beispiel 33 | R³ = Methyl | 406 | 333 |
| 35 | 1 | 3,4-Dichlorophenyl | 4-Phenoxyphenyl | 410 | 385 |
| 36 | 1 | R¹ = 3,4-Dichlorophenyl R²= 4-Tolyl, R³= Methyl | | 391 | 321 |
| 37 | 1 | 1-[4-(4'-Chlorophenyl)-naphthyl] | 4-Phenoxyphenyl | 415 | 493 |
| 38 | 1 | wie in Beispiel 37 | 4-Tolyl | 394 | 415 |
| 39 | 1 | 1-(4-Nitronaphthyl) | 2,4-Dimethoxyphenyl | 405 | 379 |
| 40 | 1 | 2-Methoxyphenyl | 4-Methoxyphenyl | 398 | 284 |
| 41 | 1 | 3-Methoxyphenyl | 4-Methoxyphenyl | 413 | 284 |
| 42 | 1 | 3,4-Dimethoxyphenyl | 4-Methoxyphenyl | 433 | 314 |
| 43 | 1 | 2-Methyl-4-methoxyphenyl | 4-Methoxyphenyl | 398 | 334 |
| 44 | 1 | 3-Methoxy-4-methylphenyl | 4-Methoxyphenyl | 420 | 334 |
| 45 | 1 | 3,5-Dimethoxyphenyl | 4-Methoxyphenyl | 412 | 314 |
| 46 | 1 | 4-Phenoxyphenyl | 4-Methoxyphenyl | 420 | 346 |
| 47 | 1 | 3-Tolyl | 4-Methoxyphenyl | 407 | 268 |
| 48 | 1 | 3,4-Dimethoxyphenyl | 4-Phenoxyphenyl | 431 | 376 |
| 49 | 1 | 3-Methoxy-4-methylphenyl | 4-Phenoxyphenyl | 421 | 360 |
| 50 | 1 | 3-Methoxy-4-methylphenyl | 3,4-Dimethoxyphenyl | 412 | 328 |
| 51 | 1 | 3,4-Dimethoxyphenyl | 4-Tolyl | 424 | 334 |
| 52 | 1 | 3-Methoxy-4-methylphenyl | 4-Tolyl | 408 | 318 |
| 53 | 1 | 4-Methoxyphenyl | 4-Tolyl | 412 | 268 |
| 54 | 1 | 4-Tolyl | 4-Tolyl | 398 | 252 |
| 55 | 1 | 3,4-Dimethylphenyl | 4-Tolyl | 389 | 266 |
| 56 | 1 | 4-Ethoxycarbonylphenyl | 4-Tolyl | 398 | 310 |
| 57 | 2 | 4-Acetamidophenyl | 4-Tolyl | 415 | 255 |
| 58 | 2 | 4-Acetamidophenyl | 4-Methoxyphenyl | 426 | 311 |
| 59 | 1 | 4-Ethoxycarbonylphenyl | 4-Methoxyphenyl | 415 | 326 |
| 60 | 1 | 2,4-Dimethylphenyl | 4-Methoxyphenyl | 399 | 282 |
| 61 | 2 | 3,4-Dimethoxyphenyl | 4-Acetamidophenyl | 436 | 306 |
| 62 | 2 | 4-Phenoxyphenyl | 4-Acetamidophenyl | 420 | 373 |
| 63 | 2 | 2-Methoxyphenyl | 4-Acetamidophenyl | 415 | 311 |
| 64 | 2 | 2,4-Dimethylphenyl | 4-Acetamidophenyl | 395 | 309 |
| 65 | 2 | 3,5-Dimethoxyphenyl | 4-Acetamidophenyl | 415 | 341 |
| 66 | 2 | 4-Acetamidophenyl | 4-Acetamidophenyl | 432 | 338 |
| 67 | 1 | 4-Phenoxyphenyl | 4-Phenoxyphenyl | 417 | 408 |
| 68 | 1 | 4-Ethoxycarbonylphenyl | 4-Phenoxyphenyl | 410 | 388 |
| 69 | 2 | 4-Phenoxyphenyl | 4-Acetamidophenyl | 423 | 373 |
| 70* | 1 | 4-Phenoxyphenyl | 4-Phenoxyphenyl | 416 | 408 |
| 71 | 2 | 3-Methoxy-4-acetamidophenyl | 4-Phenoxyphenyl | 433 | 403 |
| 72 | 2 | 3,4-Diacetamidophenyl | 4-Phenoxyphenyl | 419 | 430 |
| 73 | 2 | 2,5-Dimethyl-4-acetamidophenyl | 4-Phenoxyphenyl | 394 | 401 |
| 74 | 2 | 2,4-Dimethoxyphenyl | 3,4-Diacetamidophenyl | 410 | 398 |
| 75 | 2 | 2,4-Diacetamidophenyl | 4-Acetamidophenyl | 408 | 368 |
| 76 | 1 | R¹ = 3,4-Dimethoxyphenyl, R² = 4-Phenoxyphenyl, R³ = Methyl | | 422 | 390 |
| 77 | 1 | R¹ = 3,4-Dimethoxyphenyl, R² = 4-Methoxyphenyl, R³ = Methyl | | 425 | 328 |
| 78 | 1 | R¹ = R² = 4-Methoxyphenyl, R³ = Methyl | | 414 | 289 |
| 79 | 1 | 4-Methoxyphenyl | 4-Methoxyphenyl | 423 | 284 |
| 80 | 1 | 4-Ethoxyphenyl | 4-Ethoxyphenyl | 425 | 348 |
| 81 | 1 | 4-Ethoxyphenyl | 4-Methoxyphenyl | 423 | 334 |
| 82 | 5 | 4-Nitrophenyl | 4-Nitrophenyl | 395 | 314 |
| 83 | 1 | 4-Nitrophenyl | 4-Tolyl | 404 | 283 |
| 84 | 1 | 4-Nitrophenyl | 4-Dimethylamino | 529 | 312 |
| 85 | 2 | 3,4-Diacetamidophenyl | 4-Methoxyphenyl | 425 | 368 |
| 86 | 2 | 2,5-Dimethyl-4-acetamidophenyl | 4-Methoxyphenyl | 399 | 339 |
| 87 | 2 | 2-Methoxy-5-acetamidophenyl | 4-Methoxyphenyl | 406 | 341 |
| 88 | 2 | 3-Methoxy-4-acetamidophenyl | 4-Methoxyphenyl | 434 | 341 |
| 89 | 2 | 2-Methyl-4-acetamidophenyl | 4-Methoxyphenyl | 402 | 325 |
| 90 | 2 | 2-Methyl-4-acetamidophenyl | 4-Phenoxyphenyl | 400 | 387 |
| 91 | 2 | 3-Methoxy-4-acetamidophenyl | 3-Methoxy-4-phenoxyphenyl | 419 | 371 |
| 92 | 2 | 3-Methoxy-4-acetamidophenyl | 4-Acetamidophenyl | 419 | 368 |
| 93 | 2 | 3,4-Diacetamidophenyl | 4-Acetamidophenyl | 427 | 395 |
| 94 | 2 | 2,5-Dimethyl-4-acetamidophenyl | 4-Acetamidophenyl | 419 | 366 |
| 95 | 1 | R¹ = 3,4-Dimethoxyphenyl, R² = 4-Methoxyphenyl, R³ = Methyl | | 425 | 328 |
| 96 | 5 | Phenyl | Phenyl | 385 | 224 |
| 97 | 5 | Phenyl | 4-Methoxyphenyl | 400 | 254 |

| | | | | | |
|---|---|---|---|---|---|
| * in Beispiel 70 war X⁻ ein Tosylat-Anion. | | | | | |

### Beispiel 98

### Färbeverfahren für Polyacrylnitril

0,1 g des nach Beispiel 2 erhaltenen Farbstoffs wurden mit 2 ml Wasser unter Zusatz von wenig Essigsäure angeteigt und mit 50 ml heißem Wasser gelöst. Dann wurden 1,2 g eines Kondensationsproduktes aus Naphthalinsulfonsäure und Formaldehyd zugesetzt und mit kaltem Wasser auf 500 ml aufgefüllt.

Der pH-Wert dieser Färbeflotte wurde mit Essigsäure und Natriumacetat auf pH = 4,5 bis 5 eingestellt. In dieser Färbeflotte wurden 10 g Polyacrylnitrilfasern (Stückware) ständig in Bewegung gehalten, während in 30 min die Temperatur auf 100°C erhöht wurde. Bei Kochtemperatur wurde 60 min gefärbt, dann das Material mit kaltem Wasser gespült und bei 60 bis 70°C getrocknet. Das Material war intensiv rotstichig gelb gefärbt.

### Beispiel 99

### Färbeverfahren für holzschliffhaltiges Papier

Ein aus 60 % Holzschliff und 40 % ungebleichtem Sulfitzellstoff bestehender Trockenstoff wurde im Holländer mit soviel Wasser ausgeschlagen und bis zum Mahlgrad 40° SR gemahlen, daß der Trockengehalt etwa über 2,5 % lag. Anschließend wurde mit Wasser exakt 2,5 % Trockengehalt des Dickstoffs eingestellt. 200 g dieses Dickstoffs wurden mit 5 g einer 0,5 gew.-%igen wäßrigen Lösung des Farbstoffs erhalten nach Beispiel 4 versetzt, 5 min verrührt, 2 % Harzleim und 4 % Alaun, bezogen auf Trockenstoff, hinzugegeben und wiederum einige Minuten homogen verrührt. Dann wurde die Masse mit Wasser auf 700 ml verdünnt und hieraus in an sich bekannter Weise durch Absaugen über einem Blattbildner Papierblätter hergestellt. Sie wiesen eine intensive gelbe Färbung auf.

### Beispiel 100

Analoge Ergebnisse wie bei den Beispielen 98 und 99 wurden beim Einsatz von Farbstoffen nach Beispielen 1, 3 und 5 bis 97 erhalten.

## Patentansprüche

1. Verwendung von 4,5-Dihydro-1H-1,2,3-triazoliumverbindungen der Formel (I) 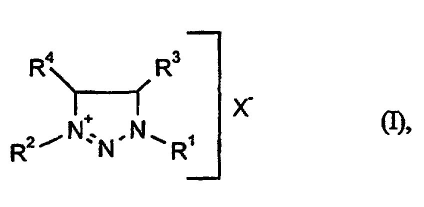 in der
R¹ und R² unabhängig voneinander C₆-C₁₄-Aryl oder einen heterocyclischen Rest mit bis zu 3 Ringen und bis zu 4 Heteroatomen aus der Reihe O, S und N bedeuten,
R³ und R⁴ unabhängig voneinander Wasserstoff, C₁-C₁₂-Alkyl, C₂-C₁₂-Alkenyl, C₄-C₈-Cycloalkyl, C₇-C₁₇-Aralkyl, C₆-C₁₄-Aryl oder Nitril bedeuten oder
R³ und R⁴ gemeinsam eine 2- bis 5-gliedrige C-Brücke bedeuten, die gegebenenfalls von bis zu zwei Sauerstoff- und/oder Stickstoffatomen unterbrochen sein kann und
X⁻ ein Anion bedeutet,
wobei alle vorhandenen Alkyl-, Alkenyl-, Cycloalkyl-, Aralkyl- und Arylreste, sowie annelierte und heterocyclische Reste gegebenenfalls mit nichtionogenen Substituenten, Carboxylgruppen, Ammoniumgruppen und/oder Pyridiniumgruppen substituiert sein können,
als Farbstoffe zum Färben und Bedrucken von kationisch färbbaren Fasern, vorzugsweise Polymerisaten und Mischpolymerisaten des Acrylnitrils und Dicyanoethylens, sowie von sauer modifizierten Fasern aus Polyamid und Polyester, von tannierten Zellulosematerialien, Papier, Seide und Leder, zur Herstellung von Schreibflüssigkeiten, Stempelflüssigkeiten, Kugelschreiberpasten und ink-jet-Tinten und im Gummidruck.

2. Verwendung nach Anspruch 1, **dadurch gekennzeichnet, daß** in Formel (I)
R¹ und R² unabhängig voneinander Phenyl, Naphthyl, Thienyl, Thiazolyl, Isothiazolyl, 1,2,4-Thiadiazolyl, 1,3,4-Thiadiazolyl, Benzthiazolyl, Benzisothiazolyl, Pyrazolyl, Imidazolyl, Benzimidazolyl, Oxazolyl, Benzoxazolyl, 1,2,3-Triazolyl, 1,2,4-Triazolyl, Indolyl oder Pyridyl bedeuten, wobei diese Reste gegebenenfalls mit bis zu 5 Resten aus der Gruppe C₁-C₈-Alkyl, C₁-C₈-Alkoxy, C₆-C₁₀-Aryloxy, C₆-C₁₀-Arylamino, C₇-C₁₂-Aralkylamino, C₁-C₈-Acylamino, C₁-C₈-Acyloxy, C₁-C₈-Monoalkylamino, Di-C₁-C₈-alkylamino, C₁-C₈-Alkoxycarbonyl, C₁-C₈-Alkoxycarbonyloxy, C₁-C₁₂-Alkamidocarbonyl, C₁-C₁₂-Alkylsulfonyl, C₆-C₁₀-Arylazo, Aminocarbonyl, Halogen, Cyano, Hydroxy, Nitro, Phenylsulfonyl, Sulfonamid, Sulfonamyl und Ureido substituiert sein können, die ihrerseits gegebenenfalls mit Hydroxy, Halogen, Cyano und/oder C₁-C₄-Alkoxy substituiert sein können,
R³ und R⁴ unabhängig voneinander jeweils Wasserstoff, gegebenenfalls durch Hydroxy, Halogen, Cyano, C₁-C₄-Alkoxy, Aminocarbonyl und/oder C ₁-C₄-Alkoxycarbonyl substituiertes C₁-C₈-Alkyl, Allyl, Cyclopentyl oder Cyclohexyl, einen gegebenenfalls durch Halogen, Cyano, C₁-C₄-Alkyl und/oder C₁-C₄-Alkoxy substituierten Benzyl-, Phenethyl-, Furyl-, Tetrahydrofurylmethyl-, Pyridyl-, Pyridylmethyl- oder Pyridylethylrest oder einen gegebenenfalls durch Halogen, Cyano, C₁-C₄-Alkyl oder C₁-C₄-Alkoxy substituierten Phenylrest bedeuten oder
R³ und R⁴ gemeinsam mit den dazwischenliegenden zwei Kohlenstoffatomen einen gegebenenfalls durch Hydroxy, Halogen, Cyano, C₁-C₄-Alkoxy und/oder Aminocarbonyl substituierten Cyclobutan-, Cyclopentan-, Cyclohexan- oder Cycloheptanring der gegebenenfalls von einem Stickstoffatom unterbrochen ist, einen gegebenenfalls durch Hydroxy, Halogen, Cyano, C₁-C₄-Alkoxy und/oder Aminocarbonyl substituierten Tetrahydropyrrolidino- oder Piperidinoring, der gegebenenfalls von einem Sauerstoffatom unterbrochen ist oder einen gegebenenfalls durch Hydroxy, Halogen, Cyano, C₁-C₄-Alkoxy und/oder Aminocarbonyl substituierten Tetrahydrofuryl- oder Tetrahydropyranylring bilden und
X⁻ ein Anion bedeutet.

3. Verwendung nach Ansprüchen 1 und 2, **dadurch gekennzeichnet, daß** die 4,5-Dihydro-1H-1,2,3-triazoliumverbindungen der Formel (I) der Formel (II) entsprechen 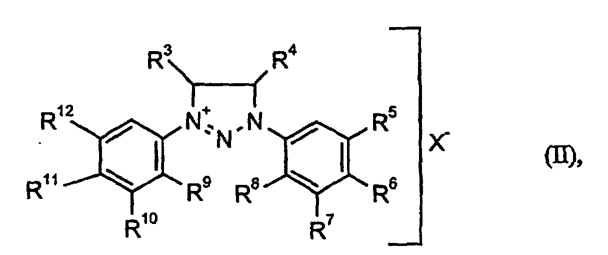 in der
R³ und R⁴ unabhängig voneinander jeweils Wasserstoff oder einen gegebenenfalls durch Hydroxy, Halogen, Cyano, C₁-C₄-Alkoxy, Aminocarbonyl und/oder C₁-C₄-Alkoxycarbonyl substituierten C₁-C₄-Alkylrest bedeuten oder
R³ und R⁴ gemeinsam mit den dazwischenliegenden zwei Kohlenstoffatomen einen gegebenenfalls durch Hydroxy, Halogen, Cyano, C₁-C₄-Alkoxy und/oder Aminocarbonyl substituierten Cyclopentan- oder Cyclohexanring bedeuten,
R⁵ bis R¹² unabhängig voneinander jeweils Wasserstoff oder einen gegebenenfalls durch Hydroxy, Halogen, Cyano und/oder C₁-C₄-Alkoxy substituierten C₁-C₄-Alkyl-, C₁-C₄-Alkoxy-, C₆-C₁₀-Aryloxy-, C₆-C₁₀-Arylamino-, C₇-C₁₂-Aralkylamino-, C₁-C₄-Acylamino-, C₁-C₄-Acyloxy-, C₁-C₄-Monoalkylamino-, Di-C₁-C₄-alkylamino, C₁-C₄-Alkoxycarbonyl-, C₁-C₄-Alkoxycarbonyloxy-, C₁-C₄-Alkamidocarbonyl-, C₁-C₄-Alkylsulfonyl- oder C₆-Arylazo-Rest, Aminocarbonyl, Halogen, Cyano, Hydroxy, Nitro, Phenylsulfonyl, Sulfonamid, Sulfonamyl oder Ureido bedeuten und
X⁻ ein Anion bedeutet

4. Verwendung nach Anspruch 3, **dadurch gekennzeichnet, daß** bei den 4,5-Dihydro-1H-1,2,3-triazoliumverbindungen der Formel (II)
R³ und R⁴ unabhängig voneinander Wasserstoff oder Methyl bedeuten,
R⁶ Wasserstoff, Methyl, Methoxy, Ethoxy, Phenoxy, Acetylamino, Dimethylamino, Diethylamino, Methoxycarbonyl, Ethoxycarbonyl, Aminocarbonyl, Chlor, Cyano oder Nitro bedeutet,
für den Fall, daß R³ und R⁴ jeweils Wasserstoff bedeuten,
R¹¹ Ethoxy, Phenoxy, Acetylamino, Dimethylamino, Diethylamino, Methoxycarbonyl, Ethoxycarbonyl, Aminocarbonyl, Chlor, Cyano oder Nitro bedeutet,
für den Fall, daß mindestens einer der Reste R³ und R⁴ von Wasserstoff verschieden ist
R¹¹ zusätzlich auch Wasserstoff, Methyl, Methoxy oder Nitro bedeuten kann,
R⁵ und R¹² und R⁷ und R¹⁰ jeweils paarweise gleich sind und Wasserstoff, Methyl, Methoxy, Ethoxy, Phenoxy, Acetylamino, Dimethylamino, Diethylamino, Methoxycarbonyl, Ethoxycarbonyl, Aminocarbonyl, Chlor, Cyano oder Nitro bedeuten,
R⁸ und R⁹ unabhängig voneinander jeweils Wasserstoff, Methyl oder Methoxy bedeuten und
X⁻ ein Anion bedeutet.

5. Verwendung nach Ansprüchen 3 und 4, **dadurch gekennzeichnet, daß** bei den 4,5-Dihydro-1H-1,2,3-triazoliumverbindungen der Formel (II)
R³, R⁵, R⁸, R⁹ und R¹² jeweils Wasserstoff bedeuten,
R⁴ Wasserstoff oder Methyl bedeutet,
R⁶ Methyl, Methoxy, Ethoxy, Phenoxy, Acetylamino, Dimethylamino, Diethylamino, Methoxycarbonyl, Ethoxycarbonyl oder Aminocarbonyl bedeutet,
für den Fall, daß R⁴ Wasserstoff bedeutet,
R¹¹ Ethoxy, Phenoxy, Acetylamino, Dimethylamino, Diethylamino, Methoxycarbonyl, Ethoxycarbonyl oder Aminocarbonyl bedeutet,
für den Fall, daß R⁴ Methyl bedeutet,
R¹¹ zusätzlich auch Wasserstoff, Methyl, Methoxy oder Nitro bedeuten kann,
R⁷ und R¹⁰ unabhängig voneinander jeweils Wasserstoff, Methyl oder Methoxy bedeuten und
X⁻ ein Anion bedeutet.

## Claims

1. Use of 4,5-dihydro-1H-1,2,3-triazolium compounds of the formula (I)
in which
R¹ and R² independently of one another denote C₆-C₁₄-aryl or a heterocyclic radical having up to 3 rings and up to 4 heteroatoms from the series consisting of O, S and N,
R³ and R⁴ independently of one another denote hydrogen, C₁-C₁₂-alkyl, C₂-C₁₂-alkenyl, C₄-C₈-cycloalkyl, C₇-C₁₇-aralkyl, C₆-C₁₄-aryl or nitrile or
R³ and R⁴ together denote a 2- to 5-membered C bridge, which can optionally be interrupted by up to two oxygen and/or nitrogen atoms, and
X⁻ denotes an anion,
wherein all the alkyl, alkenyl, cycloalkyl, aralkyl and aryl radicals and fused and heterocyclic radicals present can optionally be substituted by nonionic substituents, carboxyl groups, ammonium groups and/or pyridinium groups,
as dyestuffs for dyeing and printing cationically dyeable fibres, preferably polymers and copolymers of acrylonitrile and dicyanoethylene, and acid-modified fibres of polyamide and polyester, tannin-treated cellulose materials, paper, silk and leather, for the preparation of writing liquids, stamping liquids, ball-point pen pastes and ink-jet inks and in flexographic printing.

2. Use according to Claim 1, **characterized in that**, in formula (I),
R¹ and R² independently of one another denote phenyl, naphthyl, thienyl, thiazolyl, isothiazolyl, 1,2,4-thiadiazolyl, 1,3,4-thiadiazolyl, benzothiazolyl, benzisothiazolyl, pyrazolyl, imidazolyl, benzimidazolyl, oxazolyl, benzoxazolyl, 1,2,3-triazolyl, 1,2,4-triazolyl, indolyl or pyridyl, wherein these radicals can optionally be substituted by up to 5 radicals from the group consisting of C₁-C₈-alkyl, C₁-C₈-alkoxy, C₆-C₁₀-aryloxy, C₆-C₁₀-arylamino, C₇-C₁₂-aralkylamino, C₁-C₈-acylamino, C₁-C₈-acyloxy, C₁-C₈-monoalkylamino, di-C₁-C₈-alkylamino, C₁-C₈-alkoxycarbonyl, C₁-C₈-alkoxycarbonyloxy, C₁-C₁₂-alkamidocarbonyl, C₁-C₁₂-alkylsulphonyl, C₆-C₁₀-arylazo, aminocarbonyl, halogen, cyano, hydroxyl, nitro, phenylsulphonyl, sulphonamide, sulphonamyl and ureido, which in their turn can optionally be substituted by hydroxyl, halogen, cyano and/or C₁-C₄-alkoxy,
R³ and R⁴ independently of one another in each case denote hydrogen, C₁-C₈-alkyl, allyl, cyclopentyl or cyclohexyl which are optionally substituted by hydroxyl, halogen, cyano, C₁-C₄-alkoxy, aminocarbonyl and/or C₁-C₄-alkoxycarbonyl, a benzyl, phenethyl, furyl, tetrahydrofurylmethyl, pyridyl, pyridylmethyl or pyridylethyl radical which is optionally substituted by halogen, cyano, C₁-C₄-alkyl and/or C₁-C₄-alkoxy, or a phenyl radical which is optionally substituted by halogen, cyano, C₁-C₄-alkyl or C₁-C₄-alkoxy or
R³ and R⁴, together with the two carbon atoms in between, form a cyclobutane, cyclopentane, cyclohexane or cycloheptane ring which is optionally substituted by hydroxyl, halogen, cyano, C₁-C₄-alkoxy and/or aminocarbonyl and is optionally interrupted by a nitrogen atom, a tetrahydropyrrolidino or piperidino ring which is optionally substituted by hydroxyl, halogen, cyano, C₁-C₄-alkoxy and/or aminocarbonyl and is optionally interrupted by an oxygen atom, or a tetrahydrofuryl or tetrahydropyranyl ring which is optionally substituted by hydroxyl, halogen, cyano, C₁-C₄-alkoxy and/or aminocarbonyl and
X⁻ denotes an anion.

3. Use according to Claims 1 and 2, **characterized in that** the 4,5-dihydro-1H-1,2,3-triazolium compounds of the formula (I) correspond to the formula (II)
in which
R³ and R⁴ independently of one another in each case denote hydrogen or a C₁-C₄-alkyl radical which is optionally substituted by hydroxyl, halogen, cyano, C₁-C₄-alkoxy, aminocarbonyl and/or C₁-C₄-alkoxycarbonyl or
R³ and R⁴, together with the two carbon atoms in between, denote a cyclopentane or cyclohexane ring which is optionally substituted by hydroxyl, halogen, cyano, C₁-C₄-alkoxy and/or aminocarbonyl,
R⁵ to R¹² independently of one another in each case denote hydrogen or a C₁-C₄-alkyl, C₁-C₄-alkoxy, C₆-C₁₀-aryloxy, C₆-C₁₀-arylamino, C₇-C₁₂-aralkylamino, C₁-C₄-acylamino, C₁-C₄-acyloxy, C₁-C₄-monoalkylamino, di-C₁-C₄-alkylamino, C₁-C₄-alkoxycarbonyl, C₁-C₄-alkoxycarbonyloxy, C₁-C₄-alkamidocarbonyl, C₁-C₄-alkylsulphonyl or C₆-arylazo radical which is optionally substituted by hydroxyl, halogen, cyano and/or C₁-C₄-alkoxy, aminocarbonyl, halogen, cyano, hydroxyl, nitro, phenylsulphonyl, sulphonamide, sulphonamyl or ureido and
X⁻ denotes an anion.

4. Use according to Claim 3, **characterized in that**, in the 4,5-dihydro-1H-1,2,3-triazolium compounds of the formula (II),
R³ and R⁴ independently of one another denote hydrogen or methyl,
R⁶ denotes hydrogen, methyl, methoxy, ethoxy, phenoxy, acetylamino, dimethylamino, diethylamino, methoxycarbonyl, ethoxycarbonyl, aminocarbonyl, chlorine, cyano or nitro,
in the case where R³ and R⁴ in each case denote hydrogen,
R¹¹ denotes ethoxy, phenoxy, acetylamino, dimethylamino, diethylamino, methoxycarbonyl, ethoxycarbonyl, aminocarbonyl, chlorine, cyano or nitro,
and in the case where at least one of the radicals R³ and R⁴ is other than hydrogen
R¹¹ additionally can also denote hydrogen, methyl, methoxy or nitro,
R⁵ and R¹², and R⁷ and R¹⁰, in each case as pairs, are identical and denote hydrogen, methyl, methoxy, ethoxy, phenoxy, acetylamino, dimethylamino, diethylamino, methoxycarbonyl, ethoxycarbonyl, aminocarbonyl, chlorine, cyano or nitro,
R⁸ and R⁹ independently of one another in each case denote hydrogen, methyl or methoxy and
X⁻ denotes an anion.

5. Use according to Claims 3 and 4, **characterized in that**, in the 4,5-dihydro-1H-1,2,3-triazolium compounds of the formula (II),
R³, R⁵, R⁸, R⁹ and R¹² in each case denote hydrogen,
R⁴ denotes hydrogen or methyl,
R⁶ denotes methyl, methoxy, ethoxy, phenoxy, acetylamino, dimethylamino, diethylamino, methoxycarbonyl, ethoxycarbonyl or aminocarbonyl,
in the case where R⁴ denotes hydrogen,
R¹¹ denotes ethoxy, phenoxy, acetylamino, dimethylamino, diethylamino, methoxycarbonyl, ethoxycarbonyl or aminocarbonyl,
and in the case where R⁴ denotes methyl,
R¹¹ additionally can also denote hydrogen, methyl, methoxy or nitro,
R⁷ and R¹⁰ independently of one another in each case denote hydrogen, methyl or methoxy and
X⁻ denotes an anion.

## Revendications

1. Utilisation de dérivés de 4,5-dihydro-1H-1,2,3-triazolium répondant à la formule (I) 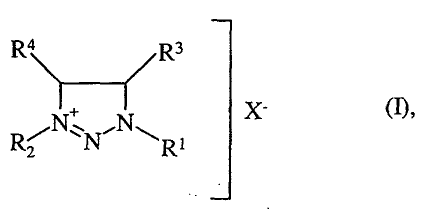 dans laquelle
R¹ et R² représentent chacun, indépendamment l'un de l'autre, un groupe aryle en C₆-C₁₄ ou un radical hétérocyclique contenant jusqu'à 3 cycles et jusqu'à 4 hétéroatomes choisis parmi O, S et N,
R³ et R⁴ représentent chacun, indépendamment l'un de l'autre, l'hydrogène, un groupe alkyle en C₁-C₁₂, alcényle en C₂-C₁₂, cycloalkyle en C₄-C₈, aralkyle en C₇-C₁₇, aryle en C₆-C₁₄ ou nitrile, ou bien
R³ et R⁴ représentent ensemble un pont de 2 à 5 chaînons carbonés qui peut être le cas échéant interrompu par 1 ou 2 atomes d'oxygène et/ou d'azote, et
X⁻ représente un anion,
tous les groupes alkyle, alcényle, cycloalkyle, aralkyle et aryle présents, de même que tous les radicaux condensés et hétérocycliques, pouvant le cas échéant porter des substituants non ioniques, des groupes carboxyle, des groupes ammonium et/ou des groupes pyridinium,
en tant que colorants pour la teinture et l'impression de fibres aptes à la teinture par colorants cationiques, de préférence des polymères et copolymères de l'acrylonitrile et du dicyanoéthylène, ainsi que de fibres en polyamide et polyester à modification acide, de matières cellulosiques tannées, du papier, de la soie et du cuir, pour la fabrication de liquides à écrire, de liquides pour tampons, de pâtes pour stylos à billes et d'encres pour dispositifs à jet d'encre, ainsi que pour l'impression du caoutchouc.

2. Utilisation selon revendication 1, **caractérisée en ce que**, dans la formule (I),
R¹ et R² représentent chacun, indépendamment l'un de l'autre, un groupe phényle, naphtyle, thiényle, thiazolyle, isothiazolyle, 1,2,4-thiadiazolyle, 1,3,4-thiadiazolyle, benzothiazolyle, benzoisothiazolyle, pyrazolyle, imidazolyle, benzimidazolyle, oxazolyle, benzoxazolyle, 1,2,3-triazolyle, 1,2,4-triazolyle, indolyle ou pyridyle, ces groupes pouvant éventuellement porter jusqu'à 5 substituants choisis parmi les groupes alkyle en C₁-C₈, alcoxy en C₁-C₈, aryloxy en C₆-C₁₀, arylamino en C₆-C₁₀, aralkylamino en C₇-C₁₂, acylamino en C₁-C₈, acyloxy en C₁-C₈, monoalkylamino en C₁-C₈, di(alkyle en C₁-C₈)amino, (alcoxy en C₁-C₈)carbonyle, (alcoxy en C₁-C₈)carbonyloxy, (alcamido en C₁-C₁₂)-carbonyle, alkylsulfonyle en C₁-C₁₂, arylazo en C₆-C₁₀, aminocarbonyle, les halogènes, les groupes cyano, hydroxy, nitro, phénylsulfonyle, sulfonamide, sulfonamyle et uréido, lesquels peuvent eux-mêmes porter le cas échéant des substituants hydroxy, halogéno, cyano et/ou alcoxy en C₁-C₄,
R³ et R⁴ représentent chacun, indépendamment l'un de l'autre, l'hydrogène, un groupe alkyle en C₁-C₈, allyle, cyclopentyle ou cyclohexyle portant éventuellement des substituants hydroxy, halogéno, cyano, alcoxy en C₁-C₄, aminocarbonyle et/ou (alcoxy en C₁-C₄)carbonyle, un groupe benzyle, phénéthyle, furyle, tétrahydrofurylméthyle, pyridyle, pyridylméthyle ou pyridyléthyle lui-même éventuellement substitué par des halogènes, des groupes cyano, alkyle en C₁-C₄ et/ou alcoxy en C₁-C₄, ou bien un groupe phényle portant éventuellement des substituants halogéno, cyano, alkyle en C₁-C₄ ou alcoxy en C₁-C₄, ou bien
R³ et R⁴ représentent ensemble et avec les deux atomes de carbone situés entre eux un noyau cyclobutane, cyclopentane, cyclohexane ou cycloheptane portant éventuellement des substituants hydroxy, halogéno, cyano, alcoxy en C₁-C₄ et/ou aminocarbonyle et qui peut le cas échéant être interrompu par un atome d'azote, un cycle tétrahydropyrrolidino ou pipéridino portant éventuellement des substituants hydroxy, halogéno, cyano, alcoxy en C₁-C₄ et/ou aminocarbonyle et qui peut le cas échéant être interrompu par un atome d'oxygène, ou un cycle tétrahydrofuryle ou tétrahydropyrannyle portant éventuellement des substituants hydroxy, halogéno, cyano, alcoxy en C₁-C₄ et/ou aminocarbonyle, et
X⁻ représente un anion.

3. Utilisation selon les revendications 1 et 2, **caractérisée en ce que** les dérivés de 4,5-dihydro-1H-1,2,3-triazolium de formule (I) répondent à la formule particulière (II) 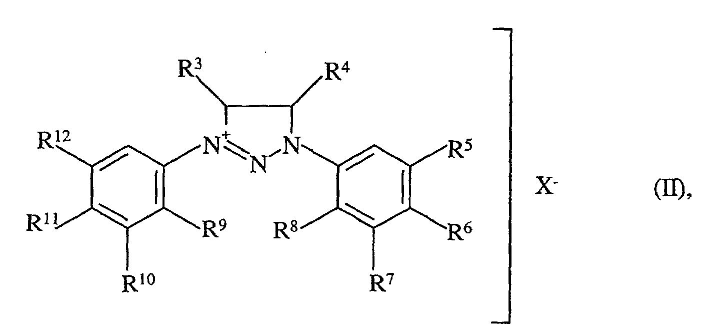 dans laquelle
R³ et R⁴ représentent chacun, indépendamment l'un de l'autre, l'hydrogène ou un groupe alkyle en C₁-C₄ portant éventuellement des substituants hydroxy, halogéno, cyano, alcoxy en C₁-C₄, aminocarbonyle et/ou (alcoxy en C₁-C₄)carbonyle, ou bien
R³ et R⁴ représentent ensemble et avec les deux atomes de carbone situés entre eux un noyau cyclopentane ou cyclohexane portant éventuellement des substituants hydroxy, halogéno, cyano, alcoxy en C₁-C₄ et/ou aminocarbonyle,
R⁵ à R¹² représentent chacun, indépendament les uns des autres, l'hydrogène ou un groupe alkyle en C₁-C₄, alcoxy en C₁-C₄, aryloxy en C₆-C₁₀, arylamino en C₆-C₁₀, aralkylamino en C₇-C₁₂, acylamino en C₁-C₄, acyloxy en C₁-C₄, monoalkylamino en C₁-C₄, di(alkyle en C₁-C₄)amino, (alcoxy en C₁-C₄)carbonyle, (alcoxy en C₁-C₄)carbonyloxy, (alcamido en C₁-C₄)carbonyle, alkylsulfonyle en C₁-C₄ ou arylazo en C₆ portant éventuellement des substituants hydroxy, halogéno, cyano et/ou alcoxy en C₁-C₄, un groupe aminocarbonyle, un halogène, un groupe cyano, hydroxy, nitro, phénylsulfonyle, sulfonamide, sulfonamyle ou uréido et
X⁻ représente un anion.

4. Utilisation selon revendication 3, **caractérisée en ce que**, pour les dérivés de 4,5-dihydro-1H-1,2,3-triazolium répondant à la formule (II),
R³ et R⁴ représentent chacun, indépendamment l'un de l'autre, l'hydrogène ou un groupe méthyle,
R⁶ représente l'hydrogène, un groupe méthyle, méthoxy, éthoxy, phénoxy, acétylamino, diméthylamino, diéthylamino, méthoxycarbonyle, éthoxycarbonyle, aminocarbonyle, le chlore, un groupe cyano ou nitro,
dans les cas où R³ et R⁴ représentent chacun l'hydrogène,
R¹¹ représente un groupe éthoxy, phénoxy, acétylamino, diméthylamino, diéthylamino, méthoxycarbonyle, éthoxycarbonyle, aminocarbonyle, le chlore, un groupe cyano ou nitro,
dans les cas où l'un au moins des symboles R³ et R⁴ a une signification autre que l'hydrogène,
R¹¹ peut en outre représenter l'hydrogène, un groupe méthyle, méthoxy ou nitro,
R⁵ et R¹² et R⁷ et R¹⁰ ont des significations identiques dans chacune de ces paires et représentent l'hydrogène, des groupes méthyle, méthoxy, éthoxy, phénoxy, acétylamino, diméthylamino, diéthylamino, méthoxycarbonyle, éthoxycarbonyle, aminocarbonyle, le chlore, des groupes cyano ou nitro,
R⁸ et R⁹ représentent chacun, indépendamment l'un de l'autre, l'hydrogène, un groupe méthyle ou méthoxy et
X⁻ représente un anion.

5. Utilisation selon les revendications 3 et 4, **caractérisée en ce que**, pour les dérivés de 4,5-dihydro-1H-1,2,3-triazolium de formule (II),
R³, R⁵, R⁸, R⁹ et R¹² représentent chacun l'hydrogène,
R⁴ représente l'hydrogène ou un groupe méthyle,
R⁶ représente un groupe méthyle, méthoxy, éthoxy, phénoxy, acétylamino, diméthylamino, diéthylamino, méthoxycarbonyle, éthoxycarbonyle ou aminocarbonyle,
dans les cas où R⁴ représente l'hydrogène,
R¹¹ représente un groupe éthoxy, phénoxy, acétylamino, diméthylamino, diéthylamino, méthoxycarbonyle, éthoxycarbonyle ou aminocarbonyle,
dans les cas où R⁴ représente un groupe méthyle,
R¹¹ peut en outre représenter l'hydrogène, un groupe méthyle, méthoxy ou nitro,
R⁷ et R¹⁰ représentent chacun, indépendamment l'un de l'autre, l'hydrogène, un groupe méthyle ou méthoxy et
X⁻ représente un anion.
